# EUROPEAN PATENT APPLICATION

(11) **EP 3 412 243 A1**
(43) Date of publication of application: **12.12.2018**
(21) Application number: 17382353.5
(22) Date of filing: 08.06.2017
(51) Int. Cl.: A61B 90/18, A61B 5/055, A61B 6/04, A61N 5/10

(54) **RIGID PVC FOAM SHEETS FOR USE IN RADIOTHERAPY AND/OR DIAGNOSIS IMAGING**

(71) Applicant: Servicio Andaluz de Salud, 41071 Sevilla (ES)
(72) Inventor: ORTIZ SEIDEL, Mónica, 41071 Spain (ES)
(74) Representative: Hoffmann Eitle

(57) **Abstract**

The present invention overcomes the deficiencies of previous treatment couches, immobilization cast fixation plates, head and neck rests or any other supportive device used in radiotherapy and provides for new supportive devices comprising PVC or PVC foam sheets, preferably rigid PVC foam sheets. By providing such materials, no metal or other features exist that can reduce the image quality and interfere with a high-energy radiation treatment beam.

## Description

### FIELD OF THE INVENTION

The present invention refers to medical field, in particular to the field of radiotherapy and diagnostic imaging. More particularly, the present invention refers to the use of PVC **(polyvinyl chloride)** or PVC foam sheets, preferably rigid PVC foam sheets, or any combination thereof, as material for immobilization devices useful in treatment by radiotherapy and diagnostic imaging.

### BACKGROUND OF THE INVENTION

Multiple fields in IMRT and optimization allow conformal dose to the target and reduced dose to the surroundings and the regions of interest. Thus we can escalate the dose to the target to achieve better tumour control with low morbidity. Orientation of multiple beams can be achieved by i) different gantry angles, ii) rotating patient's couch isocentrically. In doing so, one or more beam may pass through different materials like the treatment couch, immobilization cast fixation plate, head and neck rest or any other supportive device. Nowadays high absorption table couch tops, which required restricted gantry angles for coplanar intensity modulated radiotherapy, are no more preferred in clinical use and are being replaced by low absorption carbon fibre linac table tops, which, because of their physical properties of high strength, low density, translucence, practically no sagging at the end with weight, are more favorable for radiation treatment. In this sense, certain studies have also concluded that the attenuation of high energy photon beams by carbon fibre inserts was insignificant as compared to hardboard, copolyester and PMMA. Therefore, varieties of such carbon fibre couches and inserts are now being used in clinical practice for 3D-CRT and IMRT treatments. Original equipment manufacturers (OEM) of linacs may supply their own standard carbon fibre couch tops having different shapes and combinations of carbon fibre and foam core.

However, attenuation of the beam through carbon fibre couch has been reported. These studies were concerned with direct incidence of beam on the couch. Studies on Sinmed BV Posisert carbon fiber support for 6MV beam have reported 8.7% beam attenuation at gantry angle of 70°. EPID-based studies have also been reported on exact treatment couch insert of carbon fiber with head and neck immobilization devices and have concluded an attenuation of 15% in clinical practice. In addition, it has been reported gantry angle dependent attenuation measurements for a standard carbon fibre couch of a linac (OEM) compared with SINMED couch inserts of the order of 4.5 and 4.2% for both left and right posterior oblique beam respectively at iso-center without using any immobilization devices. Such high absorption of beam could be attributed to attenuation by the couch and immobilization devices. So when the beam passes through these materials before entering the patients, it can cause a sub-dose derived from the material's absorption, in particular for oblique incidence of beam, and an unacceptable shift in the dose distribution derived from the heterogeneous nature of the internal structure of these types of materials

We illustrate herein a novel material for the treatment couch, immobilization cast fixation plate, immobilization masks, head and neck rest or any other supportive device used in radiotherapy. Such new material provides for a low absorption that reduces the sub-dose derived from the material's absorption, in particular for oblique incidence of beam, and reduces the unacceptable shift in the dose distribution derived from the heterogeneous nature of the internal structure of these types of materials.

### BRIEF DESCRIPTION OF THE INVENTION

The present invention overcomes the deficiencies of previous treatment couches, immobilization cast fixation plates, head and neck rests or any other supportive device used in radiotherapy and provides for new supportive devices comprising PVC or PVC foam sheets, preferably rigid PVC foam sheets. By providing such materials, no metal or other features exist that can reduce the image quality and interfere with a high-energy radiation treatment beam.

The present immobilization cast fixation plates, head and neck rests or any other supportive device performs well both in diagnostic imaging and radiation therapy environments (including photon, proton and electron radiation therapy). In a preferred embodiment, the supportive devices comprising PVC or PVC foam sheets, preferably rigid PVC foam sheets, are completely free of metal in the treatment/imaging area. The treatment/imaging area is that portion of the supportive device top that can be accessed by the treatment of imaging radiation beam.

### BRIEF DESCRIPTION OF THE FIGURES

**Fig. 1****.** Attenuation measurements.

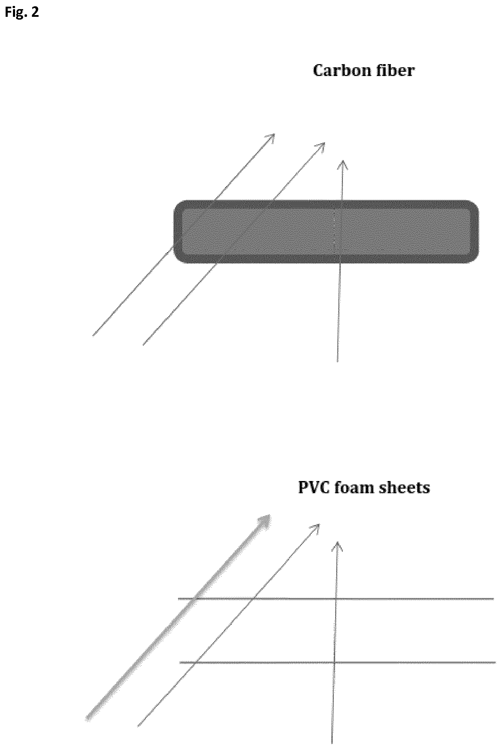

**Fig. 2****.** Comparative between carbon fibres immobilization device and PVC foam sheets immobilization devices.

### DETAILED DESCRIPTION OF THE INVENTION

A number of immobilization devices used in radiotherapy are not appropriate for use in MRI because of compatibility problems with the materials. In this sense, the dosimetry impact from devices external to the patient is a complex combination of increased skin dose, reduced tumour dose, and altered dose distribution. Although small monitor unit or dose corrections are routinely made for blocking trays, ion chamber correction factors, e.g., accounting for temperature and pressure, or tissue in-homogeneities, the dose perturbation of the treatment couch top or immobilization devices is often overlooked. These devices also increase skin dose, an effect which is also often ignored or underestimated. These concerns have grown recently due to the increased use of monolithic carbon fibre couch tops which are optimal for imaging for patient position verification but cause a significant attenuation and increased skin dose compared to the "tennis racket" style couch top they often replace. Also, arc delivery techniques have replaced stationary gantry techniques which cause a greater fraction of the dose to be delivered from posterior angles. A host of immobilization devices are available and used to increase patient positioning reproducibility, and these also have attenuation and skin dose implications which are often ignored. There are a number of reports in the known literature that illustrates the magnitude of the dosimetry effects of a wide range of devices external to the patient. There is thus a need of avoidance of high density structures during beam treatment that overcome the above mentioned difficulties.

In this sense, we herein provide a new way of manufacturing supportive devices suitable for CT/RMI image registration by using a material which is suitable for CT/RMI image registration and that at the same time provides for a low absorption that reduces the sub-dose derived from the material's absorption, in particular for oblique incidence of beam, and reduces the unacceptable shift in the dose distribution derived from the heterogeneous nature of the internal structure of these types of materials.

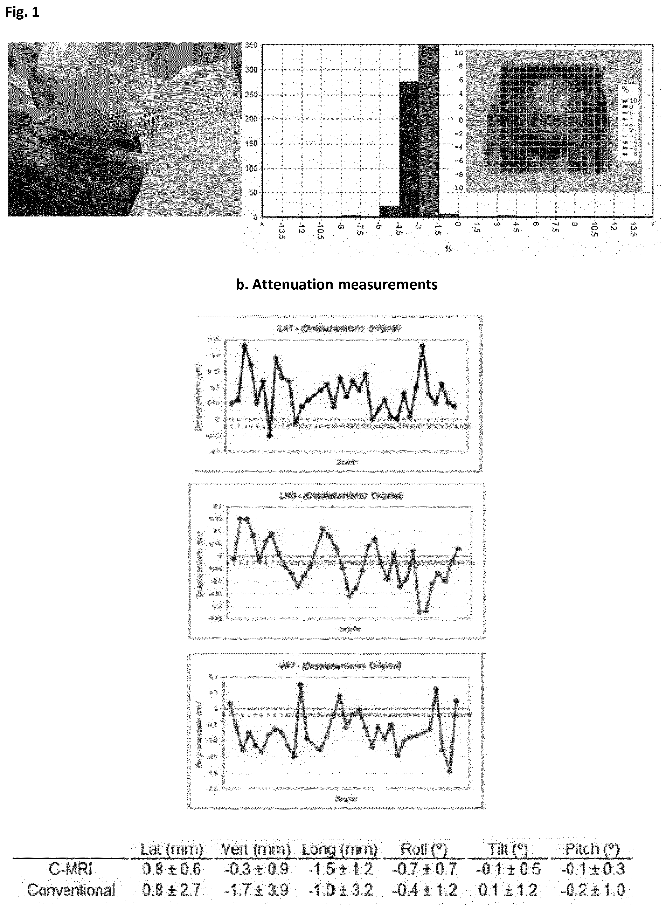

Such material are PVC or PVC foam sheets, preferably PVC foam sheets, such material was used by the authors of the present invention to manufactured immobilization equipment or devices suitable for radiotherapy (see figure 1). It is important to note that such material, PVC foam sheets, has a density of about 300 to about 700 kg/m3 (as determined by norm DIN EN ISO 1183-1), preferably from about 400 to about 700 kg/m3, more preferably from about 500 to about 700 kg/m3. Preferably, a surface hardness of from about 32 to about 44 Shore D (according to norm DIN 53 505), preferably from 37 to 44 Shore D, more preferably from about 40 to about 44 Shore D. Preferably, a coefficient of linear expansion of from about 0.04 to about 0.08 mm/(m K) (as determined by DIN EN ISO 75-2), preferably about 0.05 mm/(m K), more preferably about 0.07 mm/(m K). Preferably, a water absorption of less than 1% (as determined by DIN EN ISO 92). These types of products are commonly known as forex material.

In this sense, attenuation measurements were done using a diode array (MapCheck2, SunNuclear) inside water equivalent phantom and 6MV photons (TPR20, 10=0.685 Elekta Synergy) for orthogonal and oblique incidence of beams. Attenuation was evaluated in the area of mask fixation and in body area of frame.

Five consecutive patients with head and neck tumours were assigned to simulation with MRI compatible frame using head and shoulder masks with four fixation points (both of them made by using PVC foam sheets having a density of from about 500 to about 700 kg/m3, a thickness of about 19 mm, a surface hardness of from about 32 to about 44 Shore D (according to norm DIN 53 505), a coefficient of linear expansion of from about 0.04 to about 0.08 mm/(m K) (as determined by DIN EN ISO 75-2), and a water absorption of less than 1% (as determined by DIN EN ISO 92. Immobilization and reproducibility was improved using a customized silicone mold between patient's nose bridge and mask. Every treatment day CBCT images were acquired for treatment isocenter, and shifts in patient position were automatically measured using simulation CT as reference. Displacements in antero-posterior (Vert), cranio-caudal (Long) and medio-lateral (Lat) directions, and rotations about major axis were calculated and compared with conventional carbon fibre immobilization. A total of 150 CBCT images were acquired for CompMRI frame. A group of 30 patients with conventional board was used as control (900 CBCT images). Distribution of displacements, rotation and 3D displacements were compared between both groups.

Attenuation measurements were lower than 4% for orthogonal incidence and there was no significant difference in the attenuation measurements for oblique incidence (see figure 1). Moreover, during the planning process, we did not observe any unacceptable shift in the dose distribution. In addition, no artefacts on MRI image were observed. Reproducibility between MRI and CT simulation was better than 1 mm in all cases studied, based in direct versus automatic registration. The mean and standard deviation of shifts for the CompMRI board versus conventional board were also analysed. An analysis of variance differences using a Fisher test gives statistically significative differences between variances of two groups (p<<0.01). The distributions of the absolute displacements were similar in both groups.

Therefore, the present data shows that the C-MRI board, wherein PVC or PVC foam sheets, preferably rigid PVC foam sheets, were used, provides for low attenuation and better immobilization and reproducibility than the conventional board. Position reproducibility from MRI simulation and CT simulation was excellent. Combination of MRI compatible board with silicone fixation provided robust immobilization and can be safely used for MRI-CT registration procedures eliminating the use of deformable and complex software algorithms.

The present invention, in a first aspect of the invention, thus provides treatment couches or base frames, immobilization cast fixation plates, immobilization masks, head and neck rests or any other supportive devices used in radiotherapy comprising or consisting of PVC or PVC foam sheets, preferably rigid PVC foam sheets. By providing such materials, no metal or other features exist that can reduce the image quality and interfere with a high-energy radiation treatment beam. It is, again, important to note that such materials, in particular the PVC foam sheets used in the present invention, have an apparent density of about 300 to about 700 kg/m3 (as determined by norm DIN EN ISO 1183-1), preferably from about 400 to about 700 kg/m3, more preferably from about 500 to about 700 kg/m3. Preferably, a surface hardness of from about 32 to about 44 Shore D (according to norm DIN 53 505), preferably from 37 to 44 Shore D, more preferably from about 40 to about 44 Shore D. Preferably, a coefficient of linear expansion of from about 0.04 to about 0.08 mm/(m K) (as determined by DIN EN ISO 75-2), preferably about 0.05 mm/(m K), more preferably about 0.07 mm/(m K). Preferably, a water absorption of less than 1% (as determined by DIN EN ISO 92). These types of products are commonly known as forex material.

The present immobilization cast fixation plates, head and neck rests or any other supportive device performs well both in diagnostic imaging and radiation therapy environments (including photon, proton and electron radiation therapy). In a preferred embodiment, the supportive devices made of, comprising, or consisting of PVC or PVC foam sheets, preferably PVC foam sheets, are completely free of metal in the treatment/imaging area. The treatment/imaging area is that portion of the supportive device top that can be accessed by the treatment of imaging radiation beam.

In a second aspect of the invention, the present invention provides for the use of PVC or PVC foam sheets, preferably PVC foam sheets, for manufacturing treatment couches or base frames, immobilization cast fixation plates, immobilization masks, head and neck rests or any other supportive devices used in radiotherapy. It is noted that such PVC foam sheets have an apparent density of about 300 to about 700 kg/m3 (as determined by norm DIN EN ISO 1183-1), preferably from about 400 to about 700 kg/m3, more preferably from about 500 to about 700 kg/m3. Preferably, a surface hardness of from about 32 to about 44 Shore D (according to norm DIN 53 505), preferably from 37 to 44 Shore D, more preferably from about 40 to about 44 Shore D. Preferably, a coefficient of linear expansion of from about 0.04 to about 0.08 mm/(m K) (as determined by DIN EN ISO 75-2), preferably about 0.05 mm/(m K), more preferably about 0.07 mm/(m K). Preferably, a water absorption of less than 1% (as determined by DIN EN ISO 92).

In a third aspect of the invention, the present invention provides for the use of such PVC or PVC foam sheets, preferably rigid PVC foam sheets, in treatment regimens by radiotherapy or for use in diagnostic imaging.

It is noted that polyvinyl chloride more correctly but unusually poly(vinyl chloride), commonly abbreviated PVC, is the world's third-most widely produced synthetic plastic polymer, after polyethylene and polypropylene. PVC comes in two basic forms: rigid (sometimes abbreviated as RPVC) and flexible. The present invention refers to the rigid form of PVC. The rigid form of PVC is used in construction for pipe and in profile applications such as doors and windows. It is also used for bottles, other non-food packaging, and cards (such as bank or membership cards). It can be made softer and more flexible by the addition of plasticizers, the most widely used being phthalates. In this form, it is also used in plumbing, electrical cable insulation, imitation leather, signage, phonograph records, inflatable products, and many applications where it replaces rubber. Pure poly(vinyl chloride) is a white, brittle solid. It is insoluble in alcohol but slightly soluble in tetrahydrofuran.

It is noted that PVC foam sheets or boards, preferably rigid PVC foam sheets, used in the present invention are the commercially available and easily accessible known forex materials. It is further noted that the thickness of the rigid PVC foam sheets or boards is from 1 to 40 mm, preferably of from about 15 to 40 mm, preferably of at least 15 mm, preferably of at least about 19 mm. It is further noted that the term "about" as used herein refers to +/- 30%, preferably +/-20%, preferably +/- 10%, preferably +/- 5%, more preferably +/- 1%.

## Claims

1. A product comprising PVC or PVC foam sheets, preferably PVC foam sheets, or any combination thereof, for use in treatment regimens by radiotherapy or for use in diagnostic imaging.

2. The product for use according to claim 1, wherein the PVC foam sheets comprise a thickness of from 1 to 40 mm, preferably of from about 15 to 40 mm, preferably of at least 15 mm, preferably of at least about 19 mm., and wherein the PVC foam sheets have an apparent density of about 300 to about 700 kg/m³ (as determined by norm DIN EN ISO 1183-1).

3. The product for use according to any of claims 1 or 2, wherein the PVC foam sheets have a surface hardness of from about 32 to about 44 Shore D (according to norm DIN 53 505), optionally a coefficient of linear expansion of from about 0.04 to about 0.08 mm/(m K) (as determined by DIN EN ISO 75-2), and optionally a water absorption of less than 1% (as determined by DIN EN ISO 92).

4. The product for use according to any of claims 1 to 3, wherein such product is selected from the list consisting of treatment couches or base frames, immobilization cast fixation plates, immobilization masks, head and neck rests or any other supportive devices suitable for use in radiotherapy.

5. Treatment couches or base frames, immobilization cast fixation plates, immobilization masks, head and neck rests or any other supportive devices suitable for use in radiotherapy, comprising PVC or PVC foam sheets, preferably rigid PVC foam sheets, or any combination thereof.

6. The supportive devices of claim 5, wherein the PVC foam sheets, comprise a thickness of from 1 to 40 mm, preferably of from about 15 to 40 mm, preferably of at least 15 mm, preferably of at least about 19 mm.

7. The supportive devices of any of claims 5 or 6, wherein the PVC foam sheets have an apparent density of about 300 to about 700 kg/m³ (as determined by norm DIN EN ISO 1183-1), optionally a surface hardness of from about 32 to about 44 Shore D (according to norm DIN 53 505), optionally a coefficient of linear expansion of from about 0.04 to about 0.08 mm/(m K) (as determined by DIN EN ISO 75-2), and optionally a water absorption of less than 1% (as determined by DIN EN ISO 92).

8. Use of PVC or PVC foam sheets, preferably rigid PVC foam sheets, or any combination thereof, for manufacturing treatment couches or base frames, immobilization cast fixation plates, immobilization masks, head and neck rests or any other supportive devices suitable for use in radiotherapy.

9. The use of any of claim 7, wherein the PVC foam sheets have a thickness of from 1 to 40 mm, preferably of from about 15 to 40 mm, preferably of at least 15 mm, preferably of at least about 19 mm.

10. The use of any of claims 8 or 9, wherein the PVC foam sheets have an apparent density of about 300 to about 700 kg/m³ (as determined by norm DIN EN ISO 1183-1), optionally a surface hardness of from about 32 to about 44 Shore D (according to norm DIN 53 505), optionally a coefficient of linear expansion of from about 0.04 to about 0.08 mm/(m K) (as determined by DIN EN ISO 75-2), and optionally a water absorption of less than 1% (as determined by DIN EN ISO 92).
